# EUROPÄISCHE PATENTANMELDUNG

(11) **EP 0 867 176 A2**
(43) Veröffentlichungstag der Anmeldung: **30.09.1998**
(21) Anmeldenummer: 98104434.0
(22) Anmeldetag: 12.03.1998
(51) Int. Cl.: A61K 7/50

(54) **Duschzubereitungen mit hohem Ölgehalt**

(30) Priorität: 26.03.1997 DE 19712678
(71) Anmelder: Beiersdorf Aktiengesellschaft, 20245 Hamburg (DE)
(72) Erfinder: Lerg, Heike, 22303 Hamburg (DE); Gerber, Bozena, 22529 Hamburg (DE); Schmucker, Robert, Dr., 22457 Hamburg (DE); Stelling, Otto, 22529 Hamburg (DE)

(57) **Zusammenfassung**

Kosmetische oder dermatologische Duschzubereitungen, gekennzeichnet durch
(a) einen Gehalt von höchstens 55 Gew.-%, bezogen auf das Gesamtgewicht der Zubereitungen, eines oder mehrerer Tenside, gewählt aus der Gruppe der Trialkyl- und/oder Trialkanolaminsalze der Fettalkoholsulfate und Fettalkoholethersulfate
   sowie
(b) einen Gehalt von 30 bis 45 Gew.-%, bezogen auf das Gesamtgewicht der Zubereitungen, einer oder mehrerer Ölkomponenten, gewählt aus der Gruppe der Lipide, die ein bis drei Acylreste enthalten, die mit einem Alkohol verestert sind.
(c) einen Wassergehalt von höchstens 3,5 Gew.-%, bezogen auf das Gesamtgewicht der Zubereitungen,
(d) gegebenenfalls einen oder mehrere Emulgatoren enthaltend,
(e) gegebenenfalls weitere Tenside enthaltend,
(f) gegebenenfalls weitere kosmetische oder pharmazeutische Hilfs-, Zusatz- und/oder Wirkstoffe enthaltend.

## Beschreibung

Die vorliegende Erfindung betrifft kosmetische Reinigungszubereitungen mit einem Gehalt an Trialkyl- und/oder Trialkanolaminsalzen von Fettalkoholsulfaten und/oder Fettalkoholethersulfaten, bevorzugt für die Verwendung als Duschpräparat.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z.B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen und muß durch externe Maßnahmen regeneriert werden. Es hat daher nicht an Versuchen gefehlt, geeignete Reinigungszubereitungen zur gleichzeitigen Regeneration der Haut zu finden.

Bekannte Mittel zur Reinigung und gleichzeitigen Pflege der Haut sind z.B. Wannenbadzubereitungen, insbesondere Ölbad- oder Ölcrèmebadzubereitungen. Ihre Funktionalität beschränkt sich im allgemeinen auf die Rückfettung oder Überfettung der obersten Hautschichten. Der Stand der Technik kennt u. a. Ölbadzubereitungen verschiedener Art, wobei die Eigenschaften der Fett- oder Ölphase durch Zugabe von oberflächenaktiven Substanzen variiert werden können. Dabei können je nach Art und Menge der gewählten Bestandteile Zubereitungen formuliert werden, die auf der Badewasseroberfläche entweder spreitende Ölfilme, Öl-in-Wasser-Systeme oder auch Totalsolubilisate ergeben. Schäumende, aber auch wenig schäumende oder nicht schäumende Formulierungen sind möglich.

Die Deutsche Offenlegungsschrift 29 43 202 beschreibt Mittel mit reinigender und hautpflegender Wirkung auf der Basis von Gemischen aus Tensiden und Ölen, die bevorzugt als sogenannte Pflegeschaumbäder eingesetzt werden, wobei allerdings auch die Verwendung dieser Mittel als Duschzubereitung erwähnt wird. Die beschriebenen Zubereitungen haben einen Gehalt von 20 bis 80 Gew.-% einer wäßrigen Tensidlösung, die ihrerseits aus 85 bis 95 Gew.-% Tensid und 5 bis 15 Gew.-% Wasser besteht, sowie einen Ölgehalt von 80 bis 20 Gew.-%. Die waschaktive Komponente dieser Zubereitungen besteht aus Mono- oder Dialkylamin-, Mono- oder Dialkanolamin- oder Alkylalkanolaminsalzen von Fettalkoholschwefelsäureestern.

Die US-Patentschrift 4 371 548 beschreibt Badezusätze und Duschgele. Diese haben einen Gehalt von 20 bis 70 Gew.-% einer Tensidmischung sowie einen Ölgehalt von 20 bis 60 Gew.-%. Die Tensidmischung besteht ihrerseits zu 10 bis 90 Gew.-% aus einem oder mehreren Aminen von C₈₋₁₈-Fettalkoholsulfaten, die gegebenenfalls ethoxiliert sind, und zu 90 bis 10 Gew.-% aus einem Metall- oder Ammonium-C₈₋₁₈-Fettalkoholethersulfat.

Ein erheblicher Nachteil von Wannenbadzubereitungen ist, daß sie in sehr großer Verdünnung vorliegen, da der Inhalt einer Badewanne im Einzelfall bis zu mehreren Hundert Litern Wasser betragen kann. Dem muß durch besondere Formulierungssorgfalt bzw. Anwendung großer Mengen der zu verwendenden Ölbadzubereitung Rechnung getragen werden.

Tensidhaltige Duschzubereitungen entfalten im allgemeinen keine nennenswerte Pflegewirkung, da sie nur einen geringen Ölgehalt aufweisen. Die nicht anwendungsgerechte Nutzung einer Ölbadzubereitung als Duschpräparat ist da keine Alternative. Diese Nutzung ist unzweckmäßig, da solche Zubereitungen keinen nennenswerten Schaum entwickeln.

Eine relativ neue technische Entwicklung sind hingegen tensidhaltige Duschzubereitungen mit hohem Ölgehalt. Die Deutsche Offenlegungsschrift 44 24 210 beschreibt in diesem Zusammenhang kosmetische oder dermatologische Duschzubereitungen mit einem Tensidgehalt von höchstens 55 Gew.-% und einem Ölgehalt von mehr als 45 Gew.-%, wobei die Zubereitungen im wesentlichen wasserfrei sind. Aufgrund des hohen Ölgehalts wirken diese Zubereitungen regenerierend in bezug auf den allgemeinen Hautzustand. Sie haben dabei gleichzeitig eine gute Schaumentwicklung und eine hohe Reinigungskraft.

Überraschend und für den Fachmann nicht vorauszusehen war indes, daß Duschzubereitungen, welche einen deutlich geringeren Ölanteil haben, zu einer ebenso hohen Rückfettung der Haut führen können, wobei diese gegenüber dem Stand der Technik den Vorteil haben, noch besser zu schäumen bzw. noch besser verträglich zu sein.

Gegenstand der vorliegenden Erfindung sind daher kosmetische oder dermatologische Duschzubereitungen, gekennzeichnet durch
(a) einen Gehalt von höchstens 55 Gew.-%, bezogen auf das Gesamtgewicht der Zubereitungen, eines oder mehrerer Tenside, gewährt aus der Gruppe der Trialkyl- und/oder Trialkanolaminsalze der Fettalkoholsulfate und Fettalkoholethersulfate
   sowie
(b) einen Gehalt von 30 bis 45 Gew.-%, bezogen auf das Gesamtgewicht der Zubereitungen, einer oder mehrerer Ölkomponenten, gewährt aus der Gruppe der Lipide, die ein bis drei Acylreste enthalten, die mit einem Alkohol verestert sind,
(c) einen Wassergehalt von höchstens 3,5 Gew.-%, bezogen auf das Gesamtgewicht der Zubereitungen,
(d) gegebenenfalls einen oder mehrere Emulgatoren enthaltend,
(e) gegebenenfalls weitere Tenside enthaltend,
(f) gegebenenfalls weitere kosmetische oder pharmazeutische Hilfs-, Zusatz- und/oder Wirkstoffe enthaltend.

Die erfindungsgemäßen Zubereitungen haben eine sehr gute Schaumentwicklung, starke Reinigungskraft und entfalten eine hohe Hautpflegewirkung. Insbesondere wirken die erfindungsgemäßen Zubereitungen hautglättend, vermindern das Trockenheitsgefühl der Haut und machen die Haut geschmeidig.

Vorteilhaft weisen die erfindungsgemäß günstig zu verwendenden Fettalkoholsulfate bzw. Fettalkoholethersulfate folgende Struktur auf:

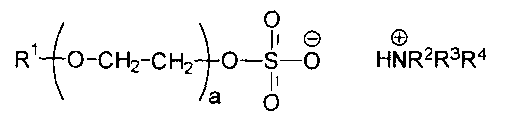

Dabei kann a Werte von 0 bis 10, vorteilhaft 1 bis 5 annehmen. R¹ wird gewählt aus der Gruppe der verzweigten und unverzweigten Alkylgruppen mit 6 bis 24 Kohlenstoffatomen. R², R³ und R⁴ werden unabhängig voneinander gewählt aus der Gruppe der verzweigten und unverzweigten Alkyl- und Hydroxyalkylreste mit 1 bis 24 Kohlenstoffatomen.

Herstellungsbedingt können die verwendeten Tenside Restmengen an physiologisch unbedenklichen, nicht umgesetzen Edukten enthalten, beispielsweise 1,2-Propylenglykol.

Bevorzugtes Fettalkoholethersulfat ist TIPA-Laurethsulfat.

Vorteilhaft werden die erfindungsgemäßen Öle aus der Gruppe der Lipide gewählt. In der biochemischen Fachsprache werden unter dem Begriff 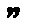 Lipide 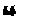 bestimmte, strukturell an sich durchaus nicht einheitliche Biomoleküle zusammengefaßt. Im ursprünglichen Sinn sind unter 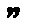 Lipiden 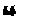 Fette zu verstehen, also Carbonsäureester des Glycerins.

Im weiteren Sinne wird in diesem Begriff eine Gruppe von in Wasser unlöslichen Molekülen verstanden, welche sich durch wenigstens einen ausgeprägt hydrophilen Molekülbereich und wenigstens einen ausgeprägt lipophilen Molekülbereich auszeichnen. Die Phosphorsäureester acylierter Glycerine, die sogenannten 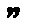 Phospholipide 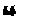 und andere Verbindungen gehören zu dieser insgesamt recht inhomogenen Gruppe chemischer Verbindungen.

Von größter Bedeutung unter den Phosphatidylcholinen sind beispielsweise die Lecithine, welche sich durch die allgemeine Struktur 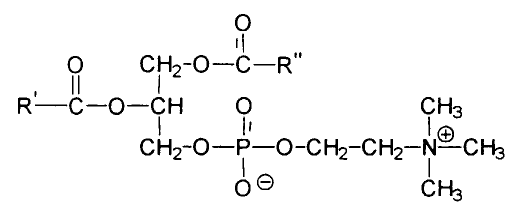 auszeichnen, wobei R^{'} und R'' typischerweise unverzweigte aliphatische Reste mit 15 oder 17 Kohlenstoffatomen und bis zu 4 cis-Doppelbindungen darstellen.

Die erfindungsgemäßen Öle werden vorzugsweise aus der Gruppe der polaren Öle, beispielsweise der Gruppe der Triglyceride oder Lecithine, und insbesondere aus der Gruppe der polaren Öle pflanzlichen Ursprungs gewählt. Nimmt man als Maß für den Polaritätsindex eines Öls bzw. einer Ölphase die Grenzflächenspannung gegenüber Wasser an, so gilt, daß die Polarität der betreffenden Ölphase umso größer ist, je niedriger die Grenzflächenspannung zwischen dieser Ölphase und Wasser ist. Die physikalische Einheit für die Grenzflächenspannung wird gewöhnlich in mN/m (Millinewton geteilt durch Meter) angegeben. Als willkürliche Grenze, unterhalb derer eine Ölphase als 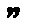 polar 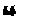 und oberhalb derer eine Ölphase als 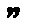 unpolar 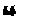 gilt, werden im allgemeinen 30 mN/m angesehen.

Erfindungsgemäß wird die Ölphase vorteilhaft, aber nicht notwendigerweise aus der Gruppe der polaren Ölkomponenten gewählt, welche eine Polarität zwischen 10 und 30 mN/m aufweisen. Vorteilhaft ist, polare pflanzliche Öle als Hauptkomponente der Ölphase zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl, Safloröl, Distelöl und Nachtkerzenöl.

Sehr vorteilhaft ist es, die erfindungsgemäßen Öle aus der Gruppe Sojaöl, Sonnenblumenöl, Weizenkeimöl, Rizinusöl und Traubenkernöl zu wählen.

Die erfindungsgemäßen Zusammensetzungen enthalten außer den vorgenannten Bestandteilen Wasser und gegebenenfalls die in der Kosmetik üblichen Zusatzstoffe, beispielsweise Emulgatoren, Verdicker, Lösungsvermittler, Parfüm, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen enthalten gegebenenfalls auch einen oder mehrere Emulgatoren. Vorteilhaft werden diese gewählt aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel R-COO-(-CH₂-CH₂-O-)ₙ -H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolether der allgemeinen Formel R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80,
- der propoxylierten Sorbitanester mit einem Propoxylierungsgrad von 3 bis 100,
- der Cholesterinpropoxylate mit einem Propoxylierungsgrad von 3 bis 100,
- der propoxylierten Triglyceride mit einem Propoxylierungsgrad von 3 bis 100,
- der Alkylethercarbonsäuren der allgemeinen Formel R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel R-COO-Xₙ-Yₘ-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Vorteilhaft ist insbesondere, Lösungsvermittler aus der Gruppe der Polyoxyethylen-Polyoxypropylen-Blockcopolymere zu wählen. Solche Blockcoploymere sind unter der Bezeichnung "Poloxamere" bekannt und zeichnen sich durch folgende Struktur aus:

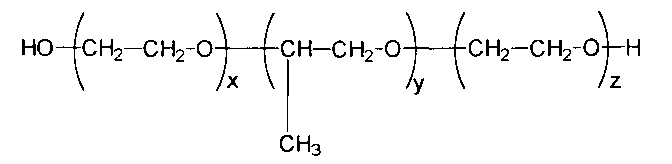

Dabei nimmt x vorteilhaft Werte zwischen 2 und 20 an. y nimmt vorteilhaft Werte zwischen 10 und 50 an. z nimmt vorteilhaft Werte zwischen 2 und 20 an.

Wenn Zubereitungen gemäß der vorliegenden Erfindung außer den erfindungsgemäßen Tensiden weitere Tenside enthalten sollen, so wird bevorzugt, deren Konzentration in bezug auf das Gewicht der Gesamtzusammensetzung nicht größer als 5 Gew.-% zu wählen.

Erfindungsgemäß können als günstige Antioxidantien alle für kosmetische und/oder dermatologische Anwendungen geeigneten oder gebräuchlichen Antioxidantien verwendet werden.

### Vorteilhaft werden die Antioxidantien gewählt aus der Gruppe

Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubiquinon und Ubiquinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung. Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die nachfolgenden Beispiele sollen die vorliegende Erfindung verdeutlichen. Die Zahlenwerte in den Beispielen bedeuten Gewichtsprozente, bezogen auf das Gesamtgewicht der jeweiligen Zubereitungen.

| **Beispiel 1:** | |
|---|---|
| TIPA-Laurethsulfat | 36 |
| Rizinusöl | 30 |
| PEG-40 Sorbitan Peroleat | 15 |
| Kokamid DEA | 10 |
| Propylenglykol | 9 |

| **Beispiel 2:** | |
|---|---|
| TIPA-Laurethsulfat | 34 |
| Rizinusöl | 30 |
| PEG-40 Sorbitan Peroleat | 15 |
| Kokamid DEA | 10 |
| Sojaöl | 9 |
| Propylenglykol | 2 |

| **Beispiel 3:** | |
|---|---|
| TIPA-Laurethsulfat | 34 |
| Rizinusöl | 30 |
| Kokamid DEA | 10 |
| PEG-40 Sorbitan Perisostearat | 10 |
| Sojaöl | 9 |
| Propylenglykol | 2 |
| Wasser | 1 |
| Parfüm, Antioxidantien, Konservierungsstoffe | q.s. |

| **Beispiel 4:** | |
|---|---|
| TIPA-Laurethsulfat | 34 |
| Rizinusöl | 30 |
| Sojaöl | 15 |
| Kokamid DEA | 10 |
| PEG-40 Sorbitan Perisostearat | 5 |
| Propylenglykol | 2 |
| Wasser | 1.8 |
| Parfüm, Antioxidantien, Konservierungsstoffe | q.s. |

| **Beispiel 5:** | |
|---|---|
| TIPA-Laurethsulfat | 36 |
| Rizinusöl | 30 |
| PEG-40 Sorbitan Peroleat | 10 |
| Kokamid DEA | 10 |
| Propylenglykol | 9 |
| Traubenkernöl | 5 |

## Patentansprüche

1. Kosmetische oder dermatologische Duschzubereitungen, gekennzeichnet durch
(a) einen Gehalt von höchstens 55 Gew.-%, bezogen auf das Gesamtgewicht der Zubereitungen, eines oder mehrerer Tenside, gewählt aus der Gruppe der Trialkyl- und/oder Trialkanolaminsalze der Fettalkoholsulfate und Fettalkoholethersulfate
sowie
(b) einen Gehalt von 30 bis 45 Gew.-%, bezogen auf das Gesamtgewicht der Zubereitungen, einer oder mehrerer Ölkomponenten, gewählt aus der Gruppe der Lipide, die ein bis drei Acylreste enthalten, die mit einem Alkohol verestert sind,
(c) einen Wassergehalt von höchstens 3,5 Gew.-%, bezogen auf das Gesamtgewicht der Zubereitungen,
(d) gegebenenfalls einen oder mehrere Emulgatoren enthaltend,
(e) gegebenenfalls weitere Tenside enthaltend,
(f) gegebenenfalls weitere kosmetische oder pharmazeutische Hilfs-, Zusatz- und/oder Wirkstoffe enthaltend.

2. Duschzubereitungen nach Anspruch 1, dadurch gekennzeichnet, daß der Alkohol, mit dem verestert wird, Glycerin, Glycerin-3-phosphat und/oder Sphingosin ist.

3. Duschzubereitungen nach Anspruch 1, dadurch gekennzeichnet, daß die Ölkomponenten gewählt werden aus der Gruppe der Öle mit einem hohen Gehalt an Triglyceriden gesättigter und/oder ungesättigter, verzweigter und/oder unverzweigter Fettsäuren und/oder aus der Gruppe der Lecithine.

4. Duschzubereitungen nach Anspruch 1, dadurch gekennzeichnet, daß die Fettalkoholsulfate bzw. Fettalkoholethersulfate folgende Struktur aufweisen: 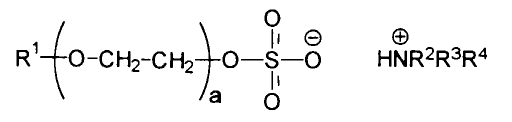 wobei a Werte von 0 bis 10, vorteilhaft 1 bis 5, annimmt und R¹ gewählt wird aus der Gruppe der verzweigten und unverzweigten Alkylgruppen mit 6 bis 24 Kohlenstoffatomen und R², R³ und R⁴ unabhängig voneinander gewählt werden aus der Gruppe der verzweigten und unverzweigten Alkyl- und Hydroxyalkylreste mit 1 bis 24 Kohlenstoffatomen.

5. Duschzubereitung nach Anspruch 4, dadurch gekennzeichnet, daß R², R³ und R⁴ folgende Struktur aufweisen:

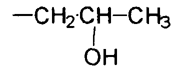

6. Duschzubereitungen nach Anspruch 1, dadurch gekennzeichnet, daß die Öle gewählt werden aus der Gruppe der polaren Öle, welche eine Polarität zwischen 10 und 30 mN/m aufweisen.

7. Duschzubereitungen nach Anspruch 1, dadurch gekennzeichnet, daß die Öle gewählt werden aus der Gruppe der Triglyceride folgender Struktur: 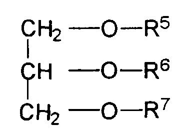 wobei R⁵, R⁶ und R⁷ unabhängig voneinander gewählt werden aus der Gruppe der verzweigten und unverzweigten, Alkylcarboxyl- bzw. Alkenylcarboxylgruppen mit 12 bis 24 Kohlenstoffatomen und gegebenenfalls ein oder mehrere aliphatische Wasserstoffatome der Alkylcarboxyl- bzw. Alkenylcarboxylgruppen durch Hydroxylgruppen substituiert sind.

8. Duschzubereitungen nach Anspruch 7, dadurch gekennzeichnet, daß R⁵, R⁶ und/oder R⁷ 16 bis 20 Kohlenstoffatome aufweisen und aus der Gruppe der einfach bis dreifach ungesättigten Carbonsäurereste gewählt werden.

9. Duschzubereitungen nach Anspruch 1, dadurch gekennzeichnet, daß das oder die Öle gewählt werden aus der Gruppe Sojaöl, Sonnenblumenöl, Weizenkeimöl, Rizinusöl, Traubenkernöl.
